# EUROPEAN PATENT SPECIFICATION

(11) **EP 2 857 047 B1**
(45) Date of publication and mention of the grant of the patent: **02.05.2018**
(21) Application number: 13797584.3
(22) Date of filing: 28.05.2013
(51) Int. Cl.: A61L 31/12, A61L 31/14, C08K 3/32, C08K 3/34

(54) **POLYMER/CERAMIC HYBRID MATERIAL**
POLYMER-/KERAMIKHYBRIDMATERIAL
MATÉRIAU HYBRIDE POLYMÈRE-CÉRAMIQUE

(30) Priority: 01.06.2012 ES 201230845
(43) Date of publication of application: 08.04.2015
(73) Proprietor: Universitat Politècnica de València, 46022 Valencia (ES)
(72) Inventor: GOMEZ RIBELLES, Jose Luis, 46022 Valencia (ES)
(74) Representative: Cueto, Sénida
(86) International application number: PCT/ES2013/070340
(87) International publication number: WO 2013/178852

(56) References cited:
- WO-A1-98/44964
- WO-A1-2009/101228
- WO-A2-2009/061908
- US-A1- 2009 304 774
- US-A1- 2011 009 327
- US-B1- 6 344 496
- S. DEVILLE ET AL: "Freezing as a Path to Build Complex Composites", SCIENCE, vol. 311, no. 5760, 27 January 2006 (2006-01-27), pages 515-518, XP055238933, US ISSN: 0036-8075, DOI: 10.1126/science.1120937
- PATTNAIK, S. ET AL.: 'Chitosan scaffolds containing silicon dioxide and zircona nanoparticles for bone tissue engineering.' INTERNATIONAL JOURNAL OF BIOLOGICAL MACROMOLECULES vol. 49, 2011, pages 1167 - 1172, XP028321993
- SHIROSAKI, Y. ET AL.: 'Synthesis and cytocompatibility of porous chitosan-silicate hybrids for tissue engineering scaffold application.' CHEMICAL ENGINEERING JOURNAL vol. 137, 2008, pages 122 - 128, XP022481092
- SILVA, S.S. ET AL.: 'Physicochemical Characterization of Novel Chitosan- Soy Protein/TEOS Porous Hybrids for Tissue Engineering Applications.' MATERIALS SCIENCE FORUM vol. 514-516, 2006, pages 1000 - 1004, XP055180230

## Description

### Field of the invention

The present invention refers to a new hybrid biodegradable material composed by a substrate or matrix of a polymeric phase and sheets of a ceramic phase obtained through a sol-gel reaction.

It is also an object of the invention obtaining said hybrid material as well as its use as a biodegradable material.

### Background of the invention

For many applications materials with apparently opposed properties are required. For example, in trauma surgery, fracture stabilization requires pieces as plates, screws, nails or rods with high stiffness but not greater than the one of the bone itself, high tensile strength to withstand high loads, impact strength, low density, and they have to be biocompatible and biodegradable. A kind of pure material, be it a metal, a ceramic or a polymer, has obvious limitations to comply with all these requirements. Metals such as titanium or stainless steel, which are not biodegradable, are currently used. Ceramics could hardly be used due to their fragility, and biodegradable polymers have a limited mechanical strength and a low elastic modulus. This is just one example in which hybrid materials, which combine the properties of one type of material and of the other one, in particular, hybrid polymer-ceramic materials are required. The very high processing temperatures of conventional ceramic materials give as a result that the combination thereof with polymers requires alternative routes of synthesis that occur at low temperatures, such as the sol-gel technique with ceramic precursors as starting materials. For example, SiO₂ networks can be formed from precursors as tetramethyl orthosilicate, TMOS, or tetraethyl orthosilicate, TEOS. The silicon atom, in these precursors, is bonded to four organic groups through oxygen atoms. The organic groups are removed by hydrolysis and condensation reactions, and the silica network is formed, starting from a liquid medium containing the precursor, some water and other solvents, and an acid or base catalyst.

There is extensive literature on the influence of the precursor chemical structure, on the kinetics of network formation and its morphology, nano-porosity, particle size, mechanical properties, and conversion degree and network homogeneity. This type of synthesis is used to produce loose micro or nano-particles or as reinforcement of a polymeric matrix, and to produce coatings. However, its fragility hardly allows to obtain materials in bulk. The properties of polymer-ceramic composite materials produced in this way is limited by the lack of interfacial adhesion between the micro- or nano-inorganic particles and the polymer matrix.

Other precursors occupy one of the four branches linked to the silicon atom in a functional organic group intended to be included into the silica network and intended to produce a covalent bond with other molecules, such as the polymer chains. The sequential or simultaneous silica network formation and the polymer chains allows for nano-hybrid composites. In the state of the art examples with chitosan or polycaprolactone matrix are described. The mechanical strength of this type of materials depends on the special form of the morphology of both phases and the adhesion therebetween.

It is known that glasses produced by living organisms have a particular impact resistance due to a particular hierarchical organization of the inorganic component and the organic component formed by proteins. Bone tissue is a good example. Thus, the present invention relates to a material wherein a ceramic network is arranged in aggregates or plates separated by thin polymer sheets. In certain compositions, the resulting set is a biodegradable material with high stiffness and strength, machinable and suitable for biomedical applications related to bone repair or bone regeneration, among other applications.

As background of the invention one can mention US patent application US2009/304774, wherein a biomaterial composed by a porous polymeric substrate which is coated by a ceramic sheet, is described. This biomaterial has many applications in the field of tissue engineering, as it can form various types of implanted devices, including bone fixation elements among them, such as biodegradable screws. In this invention, the ceramic sheet is uniformly distributed on the substrate, coating the pore wall too.

Other examples of porous biomaterials formed by a polymeric porous structure in which pore walls are coated by a ceramic sheet aimed to bone regeneration are those described in patents WO 98/44964 in which a porous bioresorbable organic polymer with hydroxyl and/or carboxylic acid groups is combined with a bioactive material, made by a sol-gel reaction, distributed within the pores of the polymer, or that described in patent US 2011/009327 that discloses a bioactive porous composite material which comprises an organic phase made of an enzymatically biodegradable organic polymer, and an inorganic phase comprising a sol-gel derived silica network, where covalent bonding is present between the organic and the inorganic phases.

Application WO9844964 discloses a biocompatible composition characterized in that it comprises a porous material of a bioresorbable organic polymer with hydroxyl- and/or carboxylic acid groups, and a bioactive material, - wherein said bioactive material is distributed within said porous material, wherein
i) said bioactive material is present as particles
   embedded in said porous material, and said porous
   material is a spongy cellulose material, and/or
ii) said bioactive material is present as a Si-gel
derived from a solution containing a silicon compound, said solution having been brought into contact with the porous material, or - wherein said porous material is a spongy cellulose material and shaped to a reservoir encompassing particles of or a gel of said bioactive material.

US2011009327 relates to a bioactive porous composite material comprising an organic phase and an inorganic phase, wherein the organic and inorganic phases are integrated and wherein the organic phase comprises an enzymatically biodegradable organic polymer and the inorganic phase comprises a sol-gel derived silica network, wherein covalent bonding is present between the organic phase and the inorganic phase and wherein the composite material comprises a source of calcium and/or strontium ions.

Finally, in the publication "Synthesis and characterization of a novel polymer-ceramic composite system for biodegradable applications" (Journal of Biomedical Material Research, Part A., Vol. 66, no. 3) an organic-inorganic biomaterial is described, suitable for generation of biodegradable bone fixation implants, such as plates, pins, screws, etc. This biomaterial includes polymer resin and ceramic fibers in the same substrate, which results in good mechanical properties due to the matched combination of the ceramic material strength and elasticity of the polymer material. As in the remaining background of the invention, the material described in this document combines polymers and ceramics forming a mixture, rather than a series of sheets joined by covalent bonds, as is the case of the present invention.

### Description of the invention

It is a first object of the invention a biodegradable hybrid material characterized in that it comprises sheets of a ceramic phase separated from each other by sheets of a polymeric organic phase to which they are covalently linked forming a compact material, wherein the sheets of ceramic phase have a thickness between hundreds of nanometers and tens of micrometers and the sheets of polymeric organic phase between them have a thickness from between 0.1 and 100 micrometers. The resulting hybrid material is a solid assembly in which organic and inorganic sheets alternate without discontinuities, The invention has as a further object the method for manufacturing said hybrid material, characterized in that it comprises:
(a) forming a polymeric organic phase by dissolving at least one precursor polymer in a solvent, followed by freezing and solvent crystallization, and subsequent removal of solvent crystals by means of sublimation or extraction, resulting in a porous structure wherein the polymeric organic phase is in the form of sheets;
(b) filling the porous structure of the polymeric organic phase with a solution of at least one inorganic precursor in the presence of at least one catalyst, forming a ceramic phase in the form of sheets covalently linked to the pore walls, i.e. to the sheets of the organic phase, forming the structure of the hybrid biodegradable material;
(c) melting and compacting the material under pressure to form a material with organic/inorganic lamellar structure. Finally, it is an object of the invention the use of a hybrid material defined in any of claims 1 to 2 for the generation of biodegradable bone fixation implants.

### Brief description of the figures

Figure 1 SEM microphotography of chitosan sponge.
Figure 2. SEM microphotography of a PCL sponge.
Figure 3. Schematic of the device for the functionalization of the sponge pore wall.

### Detailed description of the invention

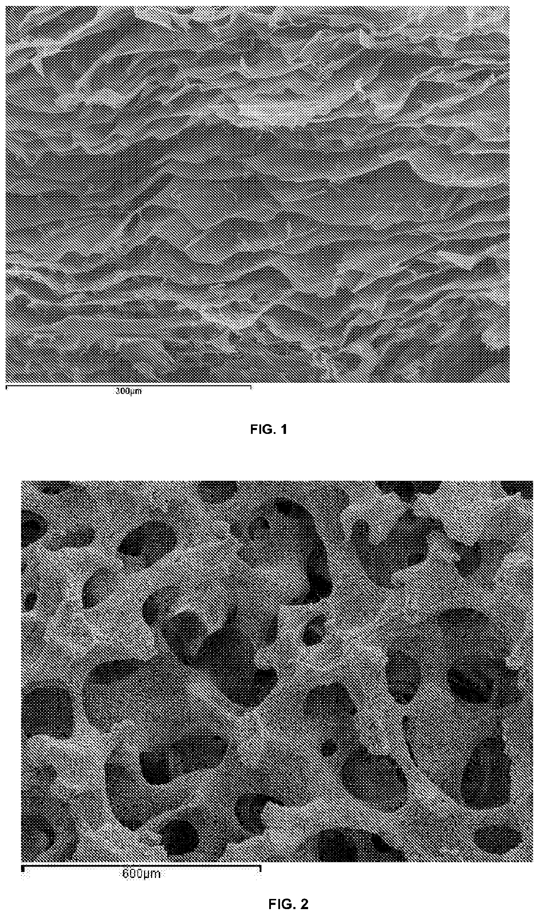

The manufacturing process of the hybrid biodegradable material object of the invention may begin by producing the organic phase in the form of porous sponge with a separation technique of solid phases. This process comprises:
(a) dissolving the polymer precursor of the organic phase in a suitable solvent, preferably selected from an aqueous solution of acid or basic pH, depending on the polymer, or at least one organic solvent preferably selected from alcohols, dioxane, or dimethyl sulfoxide, among other examples. Preferably, the solution takes place in a concentration of precursor polymer between 1 and 35% polymer by weight. Also, preferably, the precursor polymer of the organic phase is selected from a group consisting of naturally occurring polymers such as polyhydroxybutyrate, polysaccharides (chitosan, hyaluronic acid, chondroitin sulfate and others), proteins (fibrin, collagen, gelatin and others) and synthetic polymers (biodegradable polyesters such as polycaprolactone, polylactic or polyglycolic acid and their copolymers and blends, polyanhydrides etc.) and any combination thereof;
(b) freezing the solution at varying speeds. At this stage from a very low cooling (of the order of several degrees per hour) up to sudden chilling obtained for example by flash immersion in liquid nitrogen, may be used. The object is to produce crystallization of the solvent and separation of phases with the solid phase polymer. Solvent crystals can be separated by various techniques. Thus, they may be, for example, sublimated with a freeze-drying technique or can be extracted with a second solvent (preferably a slightly aggressive solvent such as aqueous solutions of suitable pH, for example, ethanol or acetone, etc. among others) at low temperature (generally, below or equal to 0 °C) temperature. The result is a porous sponge such as the one shown in Figures 1 and 2. The manufacturing process of these sponges is abundantly described in the scientific literature [M. Lebourg, J. Suay Antón, J. L. Gómez Ribelles, Eur. Polym. J. 44 (7), 2207-2218 (2008); Dunia M. García Cruz, et al. Journal of Biomedical Materials Research Part A: 95A, 1182-1193 (2010*);* Deplaine H., J. L. Gómez Ribelles, G. Gallego Ferrer, Composites Science and Technology 70, 1805-1812 (2010*);* T.C. Gamboa-Martínez, J. L. Gómez Ribelles, G. Gallego Ferrer, Journal of Bioactive and Compatible Polymers 26 (5) 464-477 (2011*),* and references therein].

The precursor (or ceramic network) of the inorganic network is prepared as a solution containing the water required for hydrolysis of the precursor, the network precursor itself or network precursors, preferably selected from a group consisting of gammaglycidoxypropyltrimethoxysilane (GPTMS), TEOS, TMOS, 3-trimetoxisillpropylmethacriyate (TSPMA), triethyl phosphate (TEP) and hydrated calcium chloride (CaCl₂.2H₂O), or mixtures thereof; and at least one acid catalyst preferably selected from acetic acid, hydrochloric acid or basic acid such as ammonium chloride.

In a particular embodiment of the invention, the solution may consist of TEOS / GPTMS / ethanol / water / acetic acid, in molar ratios 1: G: E: W: Ac with G between 0 and 10, E between 0 and 20 E, W between 0.5 and 10 and Ac between 0.01 and 0.1. In another particular relationship it may contain TEOS/TEP/CaCl₂2H₂O/MEK/ ethanol/water/hydrochloric acid, in proportions 1: T: C: M: E: W: Ac with T between 0 and 0.1, C between 0 and 0,35, M between 0 and 10, E between 0 and 1, W between 0.5 and 10 W, and Ac between 0.01 and 0.1.

In a particular way, the solution may also contain at least one binder polymer component with ability to become bonded to the inorganic network by covalent bonds and, thus, to act as a binder of the inorganic phase. Said polymer component can serve simultaneously to adjust the stiffness of the ceramic phase and can consist of a polymer preferably selected from a group consisting of chitosan, polyamides, polyacrylates or polymethacrylates polymerized in the presence of the silica precursor. To this purpose, the precursor of the inorganic network can contain a certain proportion of functional side groups ("A"). Preferably, it may contain a functional group for every atom forming the inorganic network with the capacity of chemically reacting with a "B" group of the binder polymer chains.

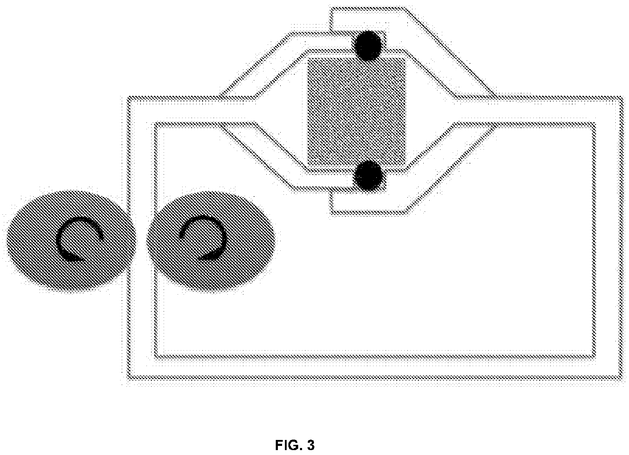

The inorganic network is formed within the pores of the sponge. The adhesion of the inorganic network with the micropore wall (micropore meanig a pore size between 0.1 and 20 micrometers) of the sponge is essential to ensure the transmission of strengths between the two phases and thus the mechanical properties of the composite. To this purpose, the process may comprise a step of wall micropore functionalization by grafting reactive groups onto it, said reacting groups being capable of reacting with the functional group "A" of the inorganic network precursor. A perfusion equipment of the needed chemical reagents may be employed to carry out the grafting as schematically shown in Figure 3. The sponge, in the shape of a membrane, thick plate, or cylindrical or prismatic bar, is set in a sample holder closing off the liquid medium which is forced to pass through the porous structure by the action of a peristaltic pump or similar equipment. After the necessary reaction time (depending on the particular reaction, the catalyst and the temperature at which it occurs) a suitable solvent (in the sense that it should not be absorbed by the polymer but it must easily penetrate in the pores) is passed, preferably being selected from ethanol, aqueous acetone solutions, etc. to remove any residual reagent. The porous structure is filled in this way with the solution of the inorganic network precursor, either using the same pumping equipment or by means of a vacuum technique. The reaction of the inorganic network formation produces highly rigid ceramic particles (high stiffness meaning an elastic modulus above 1 GPa) chemically bonded to the pore wall.

The process further includes a heating treatment (preferably at a temperature between 0 and 50 °C above the melting temperature in the case of semicrystalline polymers, or between 30 and 150 °C above the glass transition temperature in the case of amorphous polymers) that melts the matrix material and removes (usually at a pressure of between 0 and 150 MPa) the air present inside imperfections or interconnected pores and that are not filled with ceramic, under pressure.
A porous polymer sponge is obtained in this way, functionalized with ceramic particles that do not fill the pore, but that are chemically bonded to the walls, forming a sheet. Compression of the resulting porous sponge yields a laminate which is a biodegradable, machinable material, with high stiffness and strength, applicable in trauma surgery interventions, especially in applications of bone surgery.

### REFERENCE EXAMPLES

In this example, first, a chitosan sponge was prepared as the one shown in Figure 1. To this purpose the chitosan is dissolved in an aqueous solution of 0.1M acetic acid. The chitosan concentration in the solution may be between 1 and 4% by weight, what affects the porosity of the sample. The rate at which the solution is frozen (for example, by immersion in liquid nitrogen or in a freezer at -80 °C, or in a freezer at -20 °C) determines the pore size to some extent. The crystallized water is then extracted at temperatures below 0 °C with a solution of ethanol and soda while it neutralizes the chitosan making it insoluble in water. However, as the precursor solution of the silica network has an acid pH, it is necessary to crosslink the chitosan in the membrane to make it insoluble in acid medium too. To this purpose, the membrane is immersed in a solution of glutaraldehyde, genipin or another chitosan crosslinker.

The precursor solution of the ceramic network has a certain proportion of GPTMS and TEOS or TMOS, water, chitosan and hydrochloric acid as catalyst. When, once the precursor solution has filled the pores of the sponge, the sol-gel reaction takes place, the epoxy groups of GPTMS react with the amino groups of chitosan chains dissolved in the precursor solution itself as well as with the amino groups of the pore wall (in this case the pore wall does not need to be functionalized because it already contains groups capable of reacting with the silica precursor). The result is a biodegradable ceramic material with ceramic sheets of a thickness between 0.1 and 100 micrometers alternating with polymer sheets of a thickness between 1 and 100 micrometers.
2. Hybrid material formed by a biodegradable polyester (such as polycaprolactone, polylactic or polyglycolic acid or copolymers of the above mentioned ones) and silica.

The polyester sponge is analogous to the one shown in Figure 2. In this case it is prepared starting with the polymer solution in dioxane, freezing and extraction the solid dioxane with ethanol at -20 °C to generate the porous structure. Then, the sponge undergoes an aminolysis treatment in a device as the one shown in Figure 3, by circulating a 10% solution by weight of 1-6-hexanodiamine in isopropanol through the porous structure at a flow rate of 0.01 ml / min, for 2 hours, and then a mixture of water and ethanol with 70% by weight of ethanol, is passed at a flow rate of 0.1ml / min for 1 hour, and finally the samples are washed in water-ethanol stirred mixture for 24 hours. Thereby, amine groups linked by covalent bonds with polyester chains are introduced into the pore wall.

Employing the same precursor solution of the silica network as in Example 1, to which a certain chitosan amount is added (between 1 and 25% by weight), covalent bonds will be formed between the epoxy groups of GPTMS and amine groups of the pore wall and of the chitosan chains dissolved in the precursor solution. The sol-gel reaction proceeds at 40 °C for 24 hours whereby a porous material is produced, the pore walls of which are coated with silica. The sol-gel reaction fails to condense the total of the hydroxyl groups because of the temperature and time conditions. Then the temperature is raised in a mold under pressure up to 150MPa and a temperature of 80 °C. Under these conditions, the polymer sheets melt and the pore close, whereby silica sheets coating the walls meet. Thus, the temperature rise makes the silica network condensation reaction to progress, what eventually forms sheets alternating with polymer sheets in a non-porous hybrid material. This resulting hybrid material is also a biodegradable material. 3. Hybrid materials with a phase formed by a bioactive glass.

In this third example, the preparation of hybrid material was carried out starting from a process as it has been described in Examples 1 and 2 but with precursors of bioactive glass that contains triethyl phosphate (TEP) and hydrated calcium chloride (CaCl₂2H₂O), in addition to GPTMA, TEOS and/or TMOS.

## Claims

1. Hybrid biodegradable material **characterized in that** it comprises sheets of a ceramic phase separated from each other by sheets of a polymeric organic phase to which they are covalently linked forming a compact material, wherein the sheets of ceramic phase have a thickness between hundreds of nanometers and tens of micrometers and the sheets of polymeric organic phase between them have a thickness from between 0.1 and 100 micrometers.

2. Hybrid biodegradable material according to claim 1, wherein the sheets of ceramic phase have a thickness between 0.1 and 100 micrometers.

3. Process for obtaining a biodegradable hybrid material defined in any of claims 1 or 2, **characterized in that** it comprises:
(a) forming a polymeric organic phase by dissolving at least one precursor polymer in a solvent, followed by freezing and solvent crystallization, and subsequent removal of solvent crystals by means of sublimation or extraction, resulting in a porous structure wherein the polymeric organic phase is in the form of sheets;
(b) filling the porous structure of the polymeric organic phase with a solution of at least one inorganic precursor in the presence of at least one catalyst, forming a ceramic phase in the form of sheets covalently linked to the pore walls, i.e. to the sheets of the organic phase, forming the structure of the hybrid biodegradable material;
(c) melting and compacting the material under pressure to form a material with organic/inorganic lamellar structure.

4. Process according to claim 3 wherein the precursor polymer is selected from a group consisting of polyhydroxybutyrate, polysaccharides, proteins and synthetic polymers, and any combination thereof.

5. Process according to claim 3 wherein the inorganic precursor is selected from a group consisting of glycidoxypropyltrimethoxysilane -GPTMS-, tetraethoxysilane-TEOS- tetramethoxysilane -TMOS-, 3-trimetoxisilylpropylmethacrylate -TSPMA-, triethyl phosphate -TEP-, hydrated calcium chloride -CaCl₂.2H₂O- and mixtures thereof.

6. Process according to any one of claims 3 to 5, **characterized in that** it comprises an additional step of functionalization of the organic phase by adding at least one binder polymer component capable of binding to the inorganic ceramic precursor phase by formation of covalent bonds.

7. Process according to claim 6, **characterized in that** the binder polymer component is selected from a group consisting of chitosan, polyamides, polyacrylates or polymethacrylates polymerized in the presence of the silica precursor.

8. Use of a hybrid material defined in any of claims 1 to 2 for the generation of biodegradable bone fixation implants.

## Patentansprüche

1. Biologisch abbaubares Hybridmaterial, wobei es Schichten aus einer keramischen Phase umfasst, die voneinander durch Schichten einer organischen Polymerphase, mit denen sie kovalent verbunden sind, getrennt sind, und die ein kompaktes Material bilden, wobei die Schichten der keramischen Phase eine Stärke zwischen mehreren hundert Nanometern und mehreren zehn Mikrometern aufweisen und die Schichten der organischen Polymerphase untereinander eine Stärke zwischen 0,1 und 100 Mikrometern aufweisen.

2. Biologisch abbaubares Hybridmaterial nach Anspruch 1, wobei die Schichten der keramischen Phase eine Stärke zwischen 0,1 und 100 Mikrometern aufweisen.

3. Verfahren zum Erzeugen eines biologisch abbaubaren Hybridmaterials, das in einem der Ansprüche 1 oder 2 definiert wird, wobei es Folgendes umfasst:
(a) Bilden einer organischen Polymerphase durch Auflösen von zumindest einem Polymervorläufer in einem Lösungsmittel, gefolgt vom Befrosten und Kristallisieren des Lösungsmittels, und anschließendem Entfernen von Lösungsmittelkristallen mittels Sublimation oder Extraktion, wobei eine poröse Struktur entsteht, in der die organische Polymerphase in der Form von Schichten entsteht;
(b) Füllen der porösen Struktur der organischen Polymerphase mit einer Lösung von zumindest einem anorganischen Vorläufer in der Anwesenheit von zumindest einem Katalysator, wobei eine keramische Phase in der Form von Schichten gebildet wird, die mit den Porenwänden kovalent verbunden sind, d.h. mit den Schichten der organischen Phase, wobei die Struktur des biologisch abbaubaren Hybridmaterials gebildet wird;
(c) Verschmelzen und Verdichten des Materials unter Druck, um ein Material mit einer organischen/anorganischen Lamellenstruktur zu bilden.

4. Verfahren nach Anspruch 3, wobei der Polymervorläufer aus einer Gruppe bestehend aus Polyhydroxybutyraten, Polysacchariden, Proteinen und synthetischen Polymeren und jeder Kombination davon ausgewählt wird.

5. Verfahren nach Anspruch 3, wobei der anorganische Vorläufer aus einer Gruppe bestehend aus Glycidoxypropyltrimethoxysilan -GPTMS-, Tetraethoxysilan - TEOS-, Tetramethoxysilan -TMOS-, 3-Trimetoxisilylpropylmethacrylat -TSPMA-, Triethylphosphat-TEP-; hydratisiertem Calciumchlorid -CaCl₂.2H₂O- und Gemischen davon ausgewählt wird.

6. Verfahren nach einem der Ansprüche 3 bis 5, **dadurch gekennzeichnet, dass** es einen weiteren Schritt der Funktionalisierung der organischen Phase durch Hinzufügen von zumindest einer Polymerbindemittelkomponente, die sich mit der anorganischen keramischen Vorläuferphase durch Bilden von kovalenten Bindungen verbinden kann, umfasst.

7. Verfahren nach Anspruch 6, **dadurch gekennzeichnet, dass** die Polymerbindemittelkomponente aus einer Gruppe aus Chitosan, Polyamiden, Polyacrylaten oder Polyacrylaten, die in der Anwesenheit des Siliciumdioxidvorläufers polymerisiert werden, ausgewählt wird.

8. Verwendung eines in einem der Ansprüche 1 bis 2 definierten Hybridmaterials für das Erzeugen von biologisch abbaubaren Knochenfixationsimplantaten.

## Revendications

1. Matériau biodégradable hybride qui se **caractérise par le fait qu'**il comprend des couches d'une phase céramique séparées les unes des autres par des couches d'une phase organique polymérique auxquelles elles sont liées par covalence pour former un matériau compact et dont les couches de phase céramique ont une épaisseur comprise entre des centaines de nanomètres et des dizaines de micromètres et les couches de phase organique polymérique entre elles ont une épaisseur comprise entre 0,1 et 100 micromètres.

2. Matériau biodégradable hybride conformément à la revendication 1, dont les couches de phase céramique ont une épaisseur comprise entre 0,1 et 100 micromètres.

3. Procédure d'obtention du matériau hybride biodégradable défini dans les revendications 1 ou 2, **caractérisée par le fait qu'**elle comprend :
(a) La formation d'une phase organique polymérique en dissolvant au moins un précurseur polymère dans un solvant, suivi de la congélation et de la cristallisation du solvant, puis le retrait des cristaux de solvant par sublimation ou extraction, pour obtenir une structure poreuse où la phase organique polymérique se présente sous la forme de couches ;
(b) Le remplissage de la structure poreuse de la phase organique polymérique avec une solution d'au moins un précurseur inorganique en présence d'au moins un catalyseur, pour constituer une phase céramique sous la forme de couches liées par covalence aux parois des pores, à savoir aux couches de la phase organique, pour former la structure du matériau biodégradable hybride ;
(c) La fusion et le compactage du matériau sous pression pour former un matériau avec une structure lamellaire organique/inorganique.

4. Procédure, conformément à la revendication 3, dans laquelle le précurseur polymère est sélectionné dans un groupe constitué de polyhydroxybutyrate, polysaccharides, protéines et polymères synthétiques, et toutes leurs combinaisons.

5. Procédure, conformément à la revendication 3, dans laquelle le précurseur inorganique est sélectionné dans un groupe constitué de glycidoxypropyltriméthoxysilane (GPTMS), orthosilicate de tétraéthyle (TEOS) tétraméthoxysilane (TMOS), méthacrylate 3 triméthoxysilyl propyle, tétraméthoxysilane, phosphate de triéthyle (TEP), chlorure de calcium hydraté (CaCl₂.2H₂0) et leurs mélanges.

6. Procédure conformément à l'une des revendications 3 à 5, qui se **caractérise par le fait qu'**elle comprend une étape supplémentaire de fonctionnalisation de la phase organique, en ajoutant au moins un liant polymère capable de s'unir à la phase du précurseur céramique inorganique en formant des liens par covalence.

7. Procédure conformément à la revendication 6, qui se **caractérise par le fait que** le liant polymère est sélectionné dans un groupe constitué de chitosane, polyamides, polyacrylates ou polyméthacrylates polymérisés en présence du précurseur de silice.

8. Utilisation d'un matériau hybride défini dans l'une des revendications 1 ou 2 pour la génération d'implants de fixation à l'os biodégradables.
